# EUROPEAN PATENT APPLICATION

(11) **EP 3 050 517 A1**
(43) Date of publication of application: **03.08.2016**
(21) Application number: 14848070.0
(22) Date of filing: 26.05.2014
(51) Int. Cl.: A61B 10/00

(54) **OPTICAL MEASUREMENT DEVICE**

(30) Priority: 27.09.2013 JP 2013202546
(71) Applicant: FUJIFILM Corporation, Tokyo 106-8620 (JP)
(72) Inventor: MAEDA Kiyohiro, Ashigarakami-gun Kanagawa 258-8538 (JP)
(74) Representative: Klunker . Schmitt-Nilson . Hirsch
(86) International application number: PCT/JP2014/063848
(87) International publication number: WO 2015/045479

(57) **Abstract**

An optical measurement device includes a light source irradiating a test subject with first light L1 for measuring a degree of oxygen supply to a metabolic system and a photodetector receiving reflected light R1 (or transmitted light) of the first light L1 and second light R2 emitted from the test subject according to a degree of oxygen utilization by the metabolic system. In some cases, the light source irradiates a test subject with excitation light L2 such that the second light R2 is generated. The first light is, for example, light of a wavelength range that is attenuated by being partially absorbed into the test subject by passing through the test subject. Specifically, the first light is light of a wavelength range that is attenuated by being partially absorbed into oxidized hemoglobin or reduced hemoglobin contained in the test subject. The second light is, for example, fluorescence emitted from nicotinamide adenine dinucleotide or flavin adenine dinucleotide.

## Description

### BACKGROUND OF THE INVENTION

### 1. Field of the Invention

The present invention relates to an optical measurement device which measures the properties of a test subject by light.

### 2. Description of the Related Art

In the medical field, an optical measurement device including a light source which irradiates a test subject with light and a photodetector which receives light reflected from the test subject or fluorescence emitted from the test subject is widely used. For example, an optical measurement device, which measures the oxygen saturation of the blood by exploiting the light absorption of hemoglobin, is known (JP5222934B). Furthermore, an optical measurement device, which measures the amount of light emitted from nicotinamide adenine dinucleotide (NADH) in a biological body, is known. The oxygen saturation of the test subject or the amount of a metabolite such as NADH that is measured by the optical measurement device is used in various types of diagnosis.

### SUMMARY OF THE INVENTION

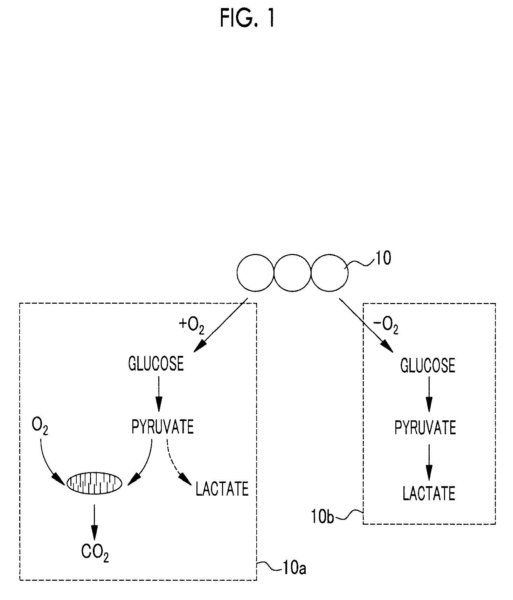

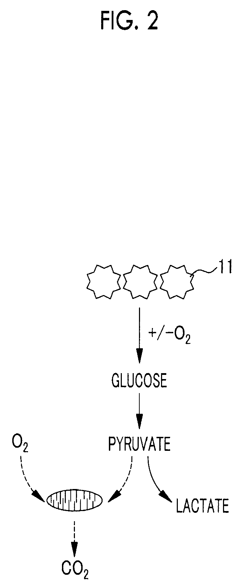

Figs. 1 and 2 are views showing the difference of the oxygen budget in metabolism between a normal tissue 10 and a cancerous tissue 11. In the normal tissue 10, the metabolism occurs in two modes according to the amount of oxygen supplied. Specifically, in case an abundant amount of oxygen is supplied (+O₂), a process called aerobic metabolism in a mode 10a occurs in the normal tissue 10. Furthermore, in case a small amount of oxygen is supplied (-O₂), a process called anaerobic metabolism, in which the oxygen utilization is inhibited, in a mode 10b occurs in the normal tissue 10. In contrast, in the cancerous tissue 11, regardless of the amount of oxygen supplied, metabolism in which the oxygen utilization is inhibited occurs at all times. Therefore, if the balance between the oxygen supply and the oxygen utilization in the metabolism of a test subject can be measured, information useful for diagnosis of cancer is obtained.

With the optical measurement device of JP5222934B, the amount of oxygen supplied can be estimated based on the oxygen saturation, but the amount of oxygen used cannot be ascertained. Therefore, the normal tissue 10 in which anaerobic metabolism occurs cannot be differentiated from the cancerous tissue 11 in which anaerobic metabolism occurs at all times.

In addition, the amount of NADH is closely related to the metabolism and increases more in the anaerobic metabolism than in the aerobic metabolism. Consequently, if the amount of light emitted from NADH is measured by the optical measurement device of JP2011-053135A, the amount of oxygen used can be estimated. However, with the optical measurement device of JP2011-053135A, the amount of oxygen supplied cannot be ascertained. As a result, the normal tissue 10 in which anaerobic metabolism occurs cannot be differentiated from the cancerous tissue 11 in which anaerobic metabolism occurs at all times.

That is, each of the optical measurement devices of JP5222934B and JP2011-053135A merely measures either the amount of oxygen supplied or the amount of oxygen used, and does not measure the balance between the oxygen utilization and the oxygen supply. Therefore, with the measurement results obtained by those optical measurement devices, cancer cannot be accurately diagnosed.

An object of the present invention is to provide an optical measurement device which can measure the balance between the oxygen utilization and the oxygen supply in the metabolism of a test subject.

An optical measurement device of the present invention includes a light source which irradiates a test subject with first light for measuring the degree of oxygen supply to a metabolic system and a photodetector which receives reflected light or transmitted light of the first light and second light emitted from the test subject according to the degree of oxygen utilization by the metabolic system.

The photodetector may be constituted with a first photodetector which is for receiving the reflected light of the first light and a second photodetector which is for receiving the second light.

The first light is, for example, light of a wavelength range that is attenuated by being partially absorbed into the test subject by passing through the test subject. Specifically, the first light is light of a wavelength range that is attenuated by being partially absorbed into oxidized hemoglobin or reduced hemoglobin contained in the test subject.

The second light is fluorescence which is emitted from the test subject, phosphorescence, or light which is generated by the Raman effect. The second light is, for example, fluorescence emitted from nicotinamide adenine dinucleotide or flavin adenine dinucleotide.

The second light may be fluorescence emitted from a substance expressed by being fused with a fluorescent protein through genetic manipulation.

A spectral filter which narrows down the wavelength range of the first light may be disposed between the light source and the test subject. Furthermore, a spectral filter which narrows down the wavelength range of the light received by the photodetector may be disposed between the test subject and the photodetector.

The light source may irradiate the test subject with excitation light such that the second light is emitted. In this case, the light source may include a first light source which radiates the first light and a second light source which radiates the excitation light. A spectral filter which narrows down the wavelength range of the excitation light may be disposed between the light source and the test subject.

The light source may irradiate the test subject with the first light and the excitation light at the same time. Furthermore, the light source may irradiate the test subject with the first light and the excitation light at different times.

It is preferable that the optical measurement device includes a signal processing portion which separates a signal generated from a substance as a measurement subject, from a light receiving signal output from the photodetector.

It is preferable that the optical measurement device includes an index value calculation portion which calculates an index value relating to the balance between the oxygen supply to the metabolic system and the oxygen utilization by the metabolic system, based on the amount of the received reflected or transmitted light of the first light and the amount of the received second light.

For example, the index value calculation portion calculates the oxygen saturation of the test subject based on the reflected or transmitted light of the first light, determines the amount of the substance emitting the second light based on the amount of the received second light, and calculates a ratio between the oxygen saturation and the amount of the substance emitting the second light as an index value.

According to the optical measurement device of the present invention, it is possible to measure the balance between the oxygen utilization and the oxygen supply in the metabolism of a test subject.

### BRIEF DESCRIPTION OF THE DRAWINGS

Fig. 1 is a view illustrating the metabolism of a normal tissue.
Fig. 2 is a view illustrating the metabolism of a cancerous tissue.
Fig. 3 is a block diagram showing the constitution of an optical measurement device.
Fig. 4 is a graph showing absorption coefficients of hemoglobin.
Fig. 5 shows an optical measurement device provided with a spectral filter disposed between a light source and a test subject.
Fig. 6 shows an optical measurement device provided with a spectral filter disposed between a test subject and a photodetector.
Fig. 7 shows an optical measurement device provided with a spectral filter disposed between a light source and a test subject and a spectral filter disposed between the test subject and a photodetector.
Fig. 8 is a graph showing absorption and emission spectra of NADH.
Fig. 9 shows an optical measurement device having a light source constituted with a first light source and a second light source.
Fig. 10 shows an optical measurement device in which each of a photodetector and a spectral filter is divided into two portions.
Fig. 11 is a view illustrating a method of multivariate analysis.
Fig. 12 shows an optical measurement device provided with a storage portion and a processing portion.
Fig. 13 shows an optical measurement device provided with an index value calculation portion.

### DESCRIPTION OF THE PREFERRED EMBODIMENTS

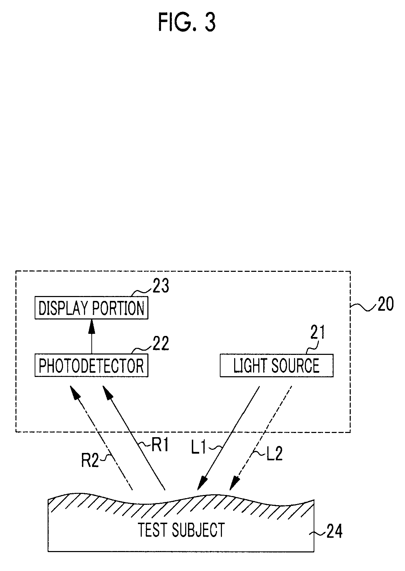

As shown in Fig. 3, an optical measurement device 20 includes a light source 21, a photodetector 22, and a display portion 23 for displaying measurement results. The light source 21 irradiates a test subject 24 with first light L1 which is for measuring a degree of oxygen supply to a metabolic system of the test subject 24. Furthermore, the photodetector 22 observes reflected light R1 of the light L1 (or transmitted light of the light L1 transmitted through the test subject 24). While passing through the test subject 24, the first light L1 is partially absorbed into a light absorbing substance in the test subject 24, attenuated, reflected (or transmitted), and received by the photodetector 22. The amount of the light absorbing substance varies depending on the amount of oxygen in the test subject 24. Accordingly, the degree of the attenuation of the first light L1 also varies depending on the amount of oxygen in the test subject 24. The degree of attenuation is observed as an index of the oxygen supply to the metabolic system of the test subject 24.

In order to observe the degree oxygen utilization in the metabolic system of the test subject 24, the photodetector 22 receives second light R2 emitted from a light emitting substance in a subject. The amount of the light emitting substance varies depending on the degree of the oxygen utilization in the metabolic system of the subject. Accordingly, the amount of the light emitted and the amount of the light received by the photodetector 22 also depend on the degree of the oxygen utilization. Therefore, the optical measurement device 20 observes the amount of received fluorescent light FL as an index of the oxygen utilization in the metabolic system of the subject. In case the light emitting substance is a fluorescent substance or a phosphorescent substance, the light source 21 radiates excitation light L2 (third light) for exciting the light emitting substance.

The test subject 24 is a biological body, and examples thereof include a portion or the entirety of an animal including a human being, samples cut off from animals, and cells.

The optical measurement device 20 is, for example, a point measurement device (a contact type or a non-contact type), a biological image measurement device, or an endoscope (a soft endoscope or a hard endoscope).

The light source 21 is, for example, a lamp of xenon, halogen, mercury, or metal halide, a light emitting diode (LED), a laser light source, or a combination of these.

The photodetector 22 is, for example, a photoelectric tube, a photomultiplier tube, a charge coupled device (CCD), or a complementary metal oxide semiconductor (CMOS) device.

The light absorbing substance is, for example, oxidized hemoglobin or reduced hemoglobin. As the amount of oxygen in the test subject 24 increases, the amount of the oxidized hemoglobin increases while the amount of the reduced hemoglobin decreases.

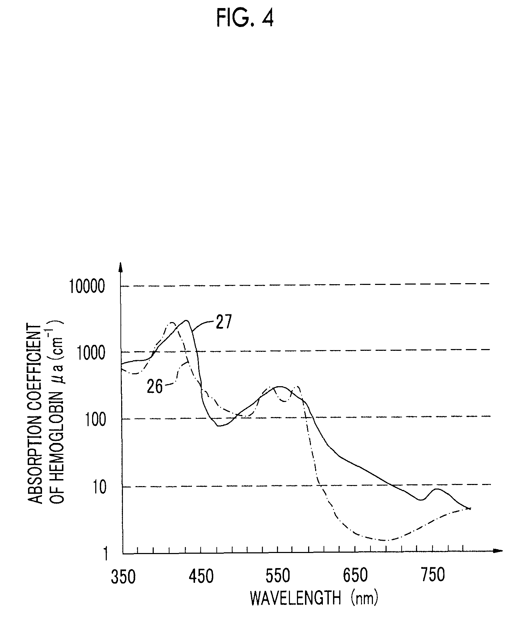

In case the light absorbing substance is the oxidized or reduced hemoglobin, the wavelength range of the first light L1 may be narrowed down to a range in which there is a great difference in light absorption between the oxidized hemoglobin and the reduced hemoglobin and a range in which there is a small difference in light absorption therebetween. As shown in Fig. 4, the range in which there is a great difference in light absorption between the oxidized hemoglobin (graph 26) and the reduced hemoglobin (graph 27) is, for example, around a wavelength of 415 nm, 435 nm, 460 nm, 515 nm, 540 nm, 560 nm, 580 nm, and 590 nm. The range in which there is a small difference in light absorption between the oxidized hemoglobin and the reduced hemoglobin is, for example, around a wavelength of 390 nm, 420 nm, 450 nm, 500 nm, 530 nm, 545 nm, 570 nm, and 585 nm. Based on the balance between the amounts of light observed in the aforementioned two regions, for example, based on a ratio or a difference between the amounts of light, the amount of oxygen may be estimated by absorbing the variation of the total amount of the hemoglobin.

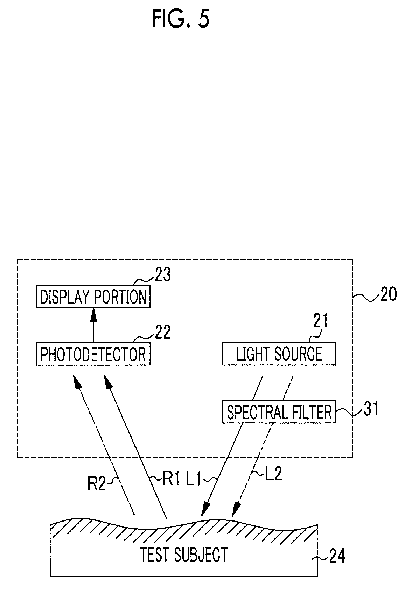

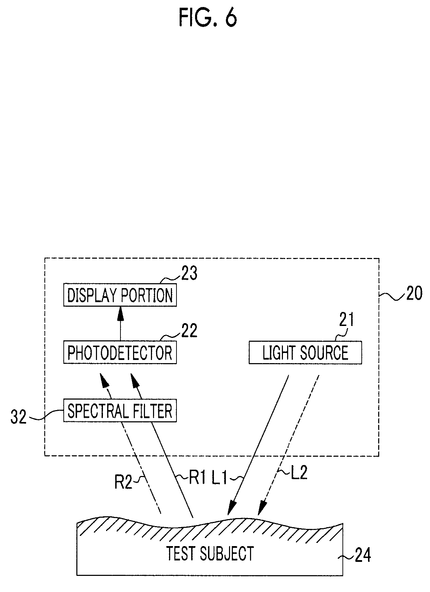

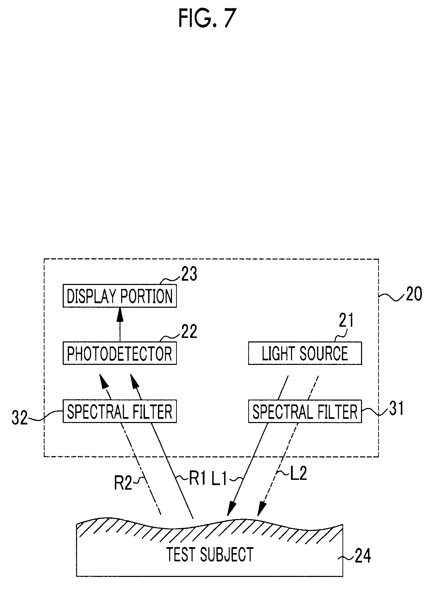

In case a broadband lighting is used as the light source 21, for example, as shown in Fig. 5, a spectral filter 31 may be disposed between the light source 21 and the test subject 24 so as to narrow down (limit) the wavelength range of the first light L1. Furthermore, as shown in Fig. 6, a spectral filter 32 may be disposed between the test subject 24 and the photodetector 22 so as to narrow down the wavelength range at the time of receiving reflected light R1 of the first light L1 or second light R2. In addition, as shown in Fig. 7, the spectral filter 31 may be disposed between the light source 21 and the test subject 24, and the spectral filter 32 may be disposed between the test subject 24 and the photodetector 22. Herein, instead of using the spectral filters 31 and 32, narrowband lighting may be used as a light source 21. It goes without saying that narrowband lighting may be used as the light source 21, and the spectral filters 31 and 32 may be additionally used in combination.

The light emitting substance is, for example, an autofluorescent substance which spontaneously exists in a biological body. The amount of nicotinamide adenine dinucleotide (NADH) as one of the autofluorescent substances increases as the oxygen utilization by the metabolic system decreases. In some cases, the amount of flavin adenine dinucleotide (FAD) varies depending on a degree of oxygen utilization by the metabolic system.

The substance whose amount or activity varies in relation to the oxygen utilization by the metabolic system may be artificially caused to emit light. For example, a protein called prolyl hydroxylase domain-containing protein 2 (PHD2), whose amount varies in relation to the oxygen utilization by the metabolic system, may be expressed by being fused with a fluorescent protein by means of manipulating the gene of PHD2.

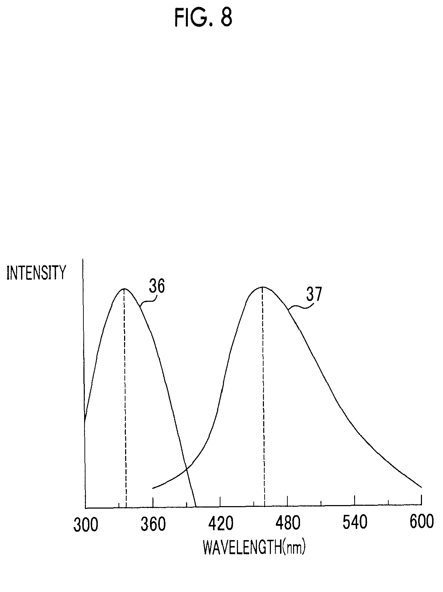

In order that the light from the light emitting substance as a measurement subject is observed differentially from another light, for example, the spectral filter 32 is preferably disposed between the test subject 24 and the photoreceiver at the time of receiving the second light R2 (see Fig. 5 or 6). For example, as shown in Fig. 8, NADH absorbs light having a wavelength of about 340 nm (graph 36) and emits fluorescence at around a wavelength of 460 nm. Accordingly, in case the light emitting substance as the measurement subject is NADH, it is preferable to use the spectral filter 32 which transmits more light at around a wavelength of 460 nm than light of other wavelength ranges.

In case the excitation light is radiated for exciting the light emitting substance, the wavelength range thereof may be narrowed down such that light in a wavelength range that is easily absorbed into the light emitting substance is used, for example. At this time, for example, broadband lighting should be used as the light source 21, and the spectral filter 31 transmitting light of a wavelength range that can be easily absorbed into the light emitting substance should be disposed between the light source 21 and the test subject 24 (see Fig. 5, 7, or 10). Furthermore, instead of providing the spectral filter 31, narrowband lighting may be used as the light source 21. It goes without saying that narrowband lighting may be used as the light source 21, and the spectral filter 31 may be additionally used in combination. For example, in case the light emitting substance as the measurement subject is NADH, it is preferable to narrow down the wavelength range such that the light having a central wavelength at around a wavelength of 320 nm to 410 nm is used (see Fig. 8).

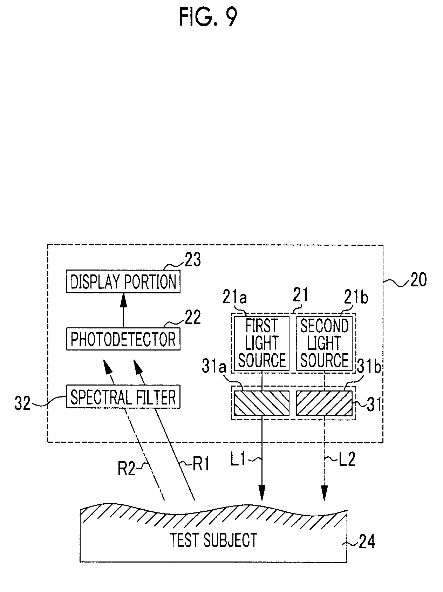

In case the light source 21 irradiates the test subject 24 with the excitation light L2 in addition to the first light L1 as shown in Fig. 9, the light source 21 can be constituted with a first light source 21a for radiating the first light L1 and a second light source 21b for radiating the excitation light L2. In case the spectral filter 31 is used, the spectral filter 31 should be divided into a spectral filter 31a which narrows down the wavelength range of the first light L1 and a spectral filter 31b which narrows down the wavelength range of the excitation light L2. The spectral filter 31a should be disposed between the first light source 21a and the test subject 24, and the spectral filter 31b should be disposed between the second light source 21b and the test subject 24. It goes without saying that only one of the spectral filter 31a and the spectral filter 31 b may be used.

In case the light source 21 is constituted with the first light source 21a and the second light source 21b, for example, a xenon lamp can be used as the first light source 21, and LED can be used as the second light source 21b. In this way, different devices can be used as the first light source 21a and the second light source 21b. It goes without saying that a xenon lamp may be used as the first light source 21a, and another xenon lamp may be used as the second light source 21b. Furthermore, for example, the same device is used as the first light source 21a and the second light source 21b. For instance, in case a xenon lamp is used as the first light source 21a, the same xenon lamp as the first light source 21a may be used as the second light source 21b, such that the light source 21 is constituted with two xenon lamps.

The first light L1 and the excitation light L2 (third light) may be radiated at the same time. In this case, the reflected light R1 of the first light L1 and the second light R2 generated by the radiation of the excitation light L2 arrive the photodetector 22 substantially at the same time. Therefore, by the combination of the spectral filter 31 and the spectral filter 32, the reflected light R1 and the second light R2 can be differentially observed.

The time of radiating the first light L1 and receiving the reflected light R1 thereof may be set to be different from the time of radiating the excitation light L2 and receiving the second light R2, such that the reflected light R1 of the first light L1 and the second light R2 are differentially observed. The temporal observation by the time-division manner described above can be realized by, for example, regulating the time during which the spectral filter 31 or 32 (or a combination thereof) functions.

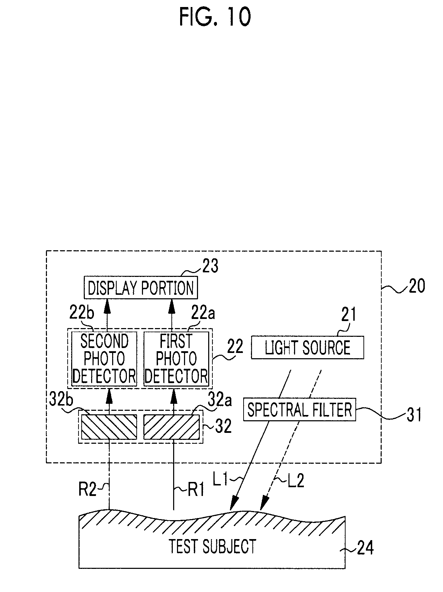

As shown in Fig. 10, the photodetector 22 may be divided into a first photodetector 22a for receiving the reflected light R1 of the first light L1 and a second photodetector 22b for receiving the second light R2. In case the spectral filter 32 is used, the spectral filter 32 should be divided into a spectral filter 32a which narrows down the wavelength range of the reflected light R1 of the first light L1 and a spectral filter 32b which narrows down the wavelength range of the second light R2. The spectral filter 32a should be disposed between the test subject 24 and the first photodetector 22a, and the spectral filter 32b should be disposed between the test subject 24 and the second photodetector 22b. It goes without saying that only one of the spectral filter 32a and the spectral filter 32b may be used. In case each of the photodetector 22 and the spectral filter 32 is divided into two portions, the reflected light R1 of the first light L1 and the second light R2 can be differentially observed.

Furthermore, the first light L1 and the excitation light L2 may be radiated at different times, such that the reflected light R1 of the first light L1 and the second light R2 arrive the photodetector 22 at different times, and the reflected light R1 of the first light L1 and the second light R2 are differentially observed. At this time, the time of radiating the first light L1 and receiving the reflected light R1 thereof may be set to be different from the time of radiating the excitation light L2 and receiving the second light R2, such that the reflected light R1 of the first light L1 and the second light R2 are easily differentiated and observed.

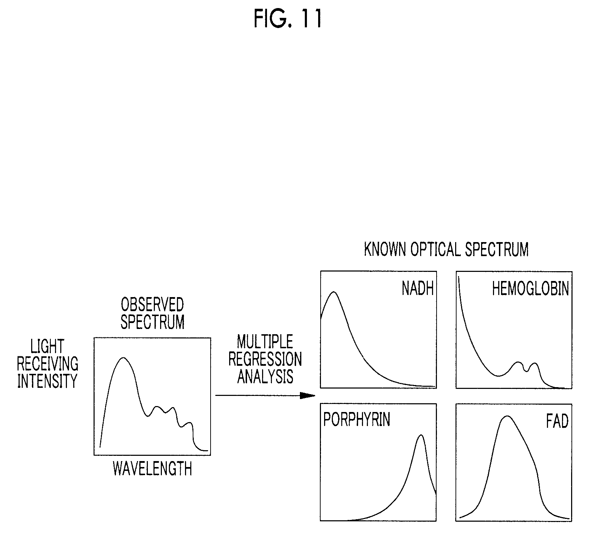

In case signals which are different from the signal resulting from the light absorption by the light absorbing substance as the measurement subject, for example, signals which result from the light absorption by the light absorbing substance not being a measurement subject or result from the light emission from the light emitting substance are mixed in the light receiving signal resulting from the reflected light R1 of the first light L1 (or in case the aforementioned signals are likely to be mixed in the light receiving signal), it is preferable to separate those signals by a method of multivariate analysis such as multiple regression analysis as shown in Fig. 11 so as to estimate the signals of light absorption by the light absorbing substance as the subject.

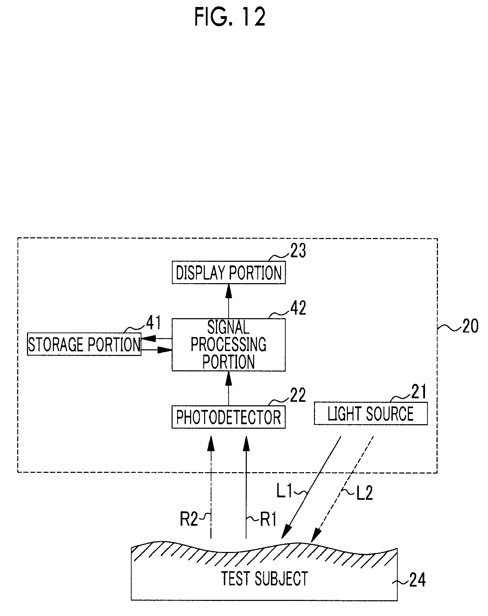

In this case, a storage portion 41 and a signal processing portion 42 should be provided as shown in Fig. 12. In the storage portion 41, data of known optical spectra such as an absorption spectrum of the light absorbing substance as a measurement subject, an absorption spectrum of the light absorbing substance not being a measurement subject, and an emission spectrum of the light emitting substance are stored in advance. By conducting fitting of the light receiving signal by using the weighted sum of the data of the known optical spectra stored in the storage portion 41, the signal processing portion 42 estimates the signal resulting from the light absorption by the light absorbing substance as the measurement subject.

The signal processing portion 42 may use a method of multivariate analysis such as non-negative matrix factorization that does not require the storage of data of known optical spectra so as to estimate the signal resulting from light absorption by the light absorbing substance as the measurement subject.

In case signals which are different from the signal resulting from the light emission from the light emitting substance as the subject, for example, signals which result from the light emission from the light emitting substance not being a subject or result from the light absorption by the light absorbing substance are mixed in the light receiving signal of the second light R2 (or in case the aforementioned signals are likely to be mixed in the light receiving signal), it is preferable to separate those signals by a method of multivariate analysis as described above so as to estimate the signals resulting from the light emission by the light emitting substance as the subject. In this case, the storage portion 41 and the signal processing portion 42 should be provided (see Fig. 11). In the storage portion 41, data such as an optical spectrum of the light emitting substance as the measurement subject, an emission spectrum of the light emitting substance which is not a subject but is likely to present in the measurement subject, and an absorption spectrum of the light absorbing substance which is likely to present in the measurement subject are stored in advance. By conducting fitting of the light receiving signal by using the weighted sum of these data, the signal processing portion 42 estimates the signal resulting from the light emission by the light emitting substance as the subject. In case the signal processing portion 42 separates the signals, which are generated from the measurement subject, from the light receiving signal of the second light R2 in the manner described above, the signal processing portion 42 may also use a method of multivariate analysis such as non-negative matrix factorization that does not require storing data in advance so as to estimate the signal resulting from the light emission by the light emitting substance as the subject.

The separation and extraction of the signal generated from the light absorbing substance as the measurement subject and the separation and extraction of the signal generated from the light emitting substance as the measurement subject can be performed during the multivariate analysis conducted once.

In the storage portion 41, for example, data of oxidized hemoglobin, reduced hemoglobin, bilimbin, myoglobin, melanin, lipofuscin, and the like are preferably stored as the data of the absorption spectrum. Furthermore, in the storage portion 41, for example, data of NADH, FAD, collagen, porphyrin, elastin, melanin, lipofuscin, and the like are preferably stored as the data of the emission spectrum. In case the light emitting substance is a fluorescent substance or a phosphorescent substance, and the emission spectrum varies with the wavelength of the excitation light, data corresponding to the wavelength of the illumination light used may be stored in the storage portion 41.

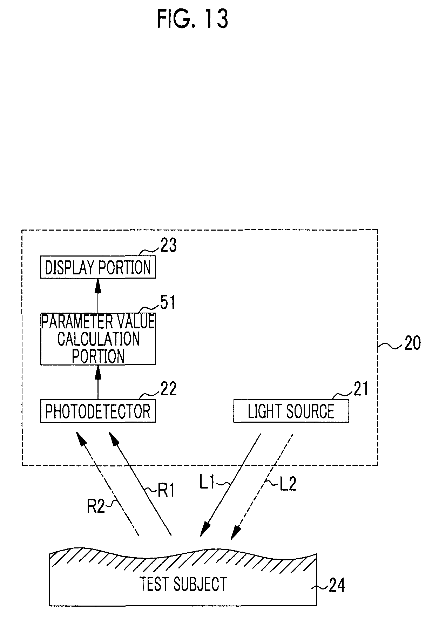

As shown in Fig. 13, the optical measurement device 20 may be provided with an index value calculation portion 51 which calculates an index value by calculating either of both of the amount of the received reflected light R1 of the first light L1 and the amount of the received second light R2. For example, the index value calculation portion 51 calculates the index value by adding an integer to the amount of the received reflected light R1 of the first light L1 or the amount of the received second light R2, subtracting an integer from the amount of the aforementioned light received, multiplying the amount of the aforementioned light received by an integer, dividing the amount of the aforementioned light received by an integer, expressing the amount of the aforementioned light received in a logarithm, an exponent, or a trigonometric function, performing the calculation for signal separation as in the signal processing portion 42, the addition, subtraction, multiplication, and division conducted between the amount of the first light received and the amount of the second light received, or a combination of these.

For example, the index value calculation portion 51 calculates, as an index value, a ratio or difference between the amount of the received reflected light R1 of the first light L1 and the amount of the received second light R2. Furthermore, a ratio or difference between a value calculated as a function of one variable of the amount of the first light received and a value calculated as a function of one variable of the amount of the second light received may be calculated as an index value. In addition, the index value calculation portion 51 may calculates a plurality of parameter values. For example, an index value calculated based on the amount of the received reflected light R1 of the first light L1 and an index value calculated based on the amount of the received second light R2 may be separately calculated. Herein, the index value calculation portion 51 may output the amount of the received reflected light R1 of the first light L1 and the amount of the received second light R2 as parameter values without particularly performing calculation.

More specifically, it is preferable that the index value calculation portion 51 calculates an index value relating to the balance between the oxygen supply to the metabolic system of the test subject 24 and the oxygen utilization by the metabolic system. For example, the oxygen saturation is calculated based on the amount of the received reflected light R1 of the first light L1, and the amount of NADH in the test subject 24 is calculated based on the amount of the received second light R2. Then, for example, a ratio therebetween (amount of NADH/oxygen saturation) is calculated as an index value. The ratio between the amount of NADH and the oxygen saturation shows the oxygen dependency in energy metabolism. Specifically, the smaller the index value, the higher the oxygen dependency in the energy metabolism. That is, it is understood that the smaller the index value, the more the aerobic metabolism predominant over the anaerobic metabolism. Accordingly, even if the oxygen saturation is low or even if the amount of NADH is great, the measured site of the test subject 24 is highly likely to be the normal tissue 10. Inversely, the greater the index value, the lower the oxygen dependency in the energy metabolism, and the more the anaerobic metabolism predominant over the aerobic metabolism. Accordingly, even if the oxygen saturation is high or even if the amount of NADH is not increasing, as long as the index value is great, the measured site of the test subject 24 is highly likely to be the cancerous tissue 11. Therefore, the ratio between the amount of NADH and the oxygen saturation is an excellent parameter for differentiating the normal tissue 10 from the cancerous tissue 11. Herein, although a ratio of amount of NADH/oxygen saturation is used as an index value, a ratio of oxygen saturation/amount of NADH may also be used as an index value.

It is preferable that the display portion 23 displays the index value output from the index value calculation portion 51 in the form of a numerical value, gray scale, color, a graph, or a combination of these. In case the photodetector 22 is an image sensor such as CCD or CMOS, the display portion 23 may display the index value in the form of an image. In this case, the index value may be displayed in the form of gray scale, color, or the like for each pixel or for each of partial region (a region consisting of a plurality of pixels) of the test subject 24.

In case an image is displayed on the display portion 23 as above, for example, if there is a plurality of parameter values consisting of parameter values relating to each of the reflected light R1 of the first light L1 and the second light R2, when a user designates a place in the image, the index value in the place may be displayed in the form of a numerical value, gray scale, color, or a graph that pops out. Furthermore, the images showing the respective parameter values may be displayed in a line or displayed by being temporally switched with each other. The images may be automatically switched at a predetermined timing or switched based on the instruction from the user.

In case a plurality of parameter values is calculated, a lookup table associating those parameter values with the displayed gray scale or the color in the display may be preset such that display is performed based on the lookup table. For example, the lookup table may be a correspondence table in which the plurality of parameter values is associated with the intensity of channels of red, green, and blue of the display. The correspondence table is, for example, linear correspondence expressed in a matrix.

The display portion 23 may display the history of the index value in the form of a numerical value, gray scale, color, a graph, or a combination of these. Therefore, the display portion 23 may be provided with a device (or a storage portion) which stores the index values of the past.

The optical measurement device 20 can be used for diagnosis of cancer, for example. In addition, for example, in developing a drug, the optical measurement device 20 can be used for evaluating drug efficacy or for obtaining a guideline regarding in what way the compound structure of the drug should be changed. In these cases, for improving the convenience of the user, it is preferable that the display portion 23 displays determination results showing whether or not the test subject 24 has cancer, the probability thereof, a degree of malignancy of cancer, the type of cancer, the prognosis prediction, the potency of drug efficacy, a suggested drug, and the like.

Herein, the optical measurement device 20 measures the amount of a substance correlating to the oxygen utilization in the metabolic system of the test subject 24 based on the amount of the received second light R2 generated by the radiation of the excitation light L2. However, the second light R2 includes not only fluorescence or phosphorescence but also light generated by the Raman effect. That is, the optical measurement device 20 can measure the amount of a substance correlating to the oxygen utilization in the metabolic system of the test subject 24 by the Raman spectroscopy.

### Explanation of References

10: normal tissue
11: cancerous tissue
20: optical measurement device
21: light source
22: photodetector
23: display portion
41: storage portion
42: processing portion
51: parameter value calculation portion

## Claims

1. An optical measurement device comprising:
a light source irradiating a test subject with first light which is for measuring a degree of oxygen supply to a metabolic system; and
a photodetector receiving reflected light or transmitted light of the first light and second light which is emitted from the test subject according to a degree of oxygen utilization by the metabolic system.

2. The optical measurement device according to claim 1,
wherein the photodetector includes a first photodetector for receiving the reflected light of the first light and a second photodetector for receiving the second light.

3. The optical measurement device according to claim 1 or 2,
wherein the first light is light of a wavelength range that is attenuated by being partially absorbed into the test subject by passing through the test subject.

4. The optical measurement device according to claim 3,
wherein the first light is light of a wavelength range that is attenuated by being partially absorbed into oxidized hemoglobin or reduced hemoglobin contained in the test subject.

5. The optical measurement device according to any one of claims 1 to 4,
wherein the second light is fluorescence emitted from the test subject, phosphorescence, or light generated by the Raman effect.

6. The optical measurement device according to claim 5,
wherein the second light is fluorescence emitted from an autofluorescent substance contained in the test subject.

7. The optical measurement device according to claim 6,
wherein the second light is fluorescence emitted from nicotinamide adenine dinucleotide or flavin adenine dinucleotide.

8. The optical measurement device according to claim 5,
wherein the second light is fluorescence emitted from a substance which is expressed by being fused with a fluorescent protein through genetic manipulation.

9. The optical measurement device according to any one of claims 1 to 8, further comprising:
a spectral filter, which narrows down the wavelength range of the first light, between the light source and the test subject.

10. The optical measurement device according to any one of claims 1 to 9, further comprising:
a spectral filter, which narrows down the wavelength range of the light received by the photodetector, between the test subject and the photodetector.

11. The optical measurement device according to any one of claims 1 to 10,
wherein the light source irradiates the test subject with excitation light such that the second light is emitted.

12. The optical measurement device according to claim 11,
wherein the light source includes a first light source for radiating the first light and a second light source for radiating the excitation light.

13. The optical measurement device according to claim 11 or 12, further comprising:
a spectral filter, which narrows down a wavelength range of the excitation light, between the light source and the test subject.

14. The optical measurement device according to any one of claims 11 to 13,
wherein the light source irradiates the test subject with the first light and the excitation light at the same time.

15. The optical measurement device according to any one of claims 11 to 13,
wherein the light source irradiates the test subject with the first light and the excitation light at different times.

16. The optical measurement device according to any one of claims 1 to 15, further comprising:
a signal processing portion separating a signal, which is generated from a substance as a measurement subject, from a light receiving signal output from the photodetector.

17. The optical measurement device according to any one of claims 1 to 16, further comprising:
an index value calculation portion calculating an index value relating to the balance between the oxygen supply to the metabolic system and the oxygen utilization by the metabolic system based on the amount of the received reflected or transmitted light of the first light and the amount of the received second light.

18. The optical measurement device according to claim 17,
wherein the index value calculation portion calculates oxygen saturation of the test subject based on the reflected or transmitted light of the first light, determines the amount of the substance emitting the second light based on the amount of the received second light, and calculates a ratio between the oxygen saturation and the amount of the substance emitting the second light as the index value.
